Europäisches Patentamt

European Patent Office

Office européen des brevets

(19)

(11) **EP 0 810 230 A2**

(12) **EUROPÄISCHE PATENTANMELDUNG**

(43) Veröffentlichungstag:
03.12.1997 Patentblatt 1997/49

(51) Int. Cl.⁶: $C07K\ 16/10$, $G01N\ 33/577$

(21) Anmeldenummer: 97108824.0

(22) Anmeldetag: 02.06.1997

(84) Benannte Vertragsstaaten:
**DE ES FR GB IT**

(30) Priorität: 31.05.1996 DE 19622088

(71) Anmelder:
**BOEHRINGER MANNHEIM GMBH**
**68305 Mannheim (DE)**

(72) Erfinder:
• **Ofenloch-Hähnle, Beatus, Dr.**
**82398 Polling (DE)**

• **Faatz, Elke, Dr.**
**82386 Huglfing (DE)**
• **Höb, Eva, Dr.**
**81476 München (DE)**
• **Borgya, Anneliese**
**82327 Tutzing (DE)**
• **Hübner-Parajsz, Christa, Dr.**
**82327 Tutzing (DE)**

(74) Vertreter:
**Weiss, Wolfgang, Dipl.-Chem. Dr. et al**
**Postfach 86 08 20**
**81635 München (DE)**

(54) **Anti-HIV-Antikörper als Kontrollproben**

(57) Die vorliegende Erfindung betrifft neue monoklonale Antikörper gegen Antigene aus HIV, insbesondere gegen das Antigen gp41 aus HIV-Subtyp 0 und das Antigen gp32 aus HIV II. Weiterhin betrifft die vorliegende Erfindung die Verwendung der Antikörper in einem immunologischen Verfahren, insbesondere als Kontrollproben in Testkits zum Nachweis von Anti-HIV-Antikörpern und zur Qualitätskontrolle bei der Herstellung von HIV-Antigenen für solche Testkits. Außerdem werden Testkits zum Nachweis von Antikörpern gegen mikrobielle Erreger, insbesondere virale Erreger offenbart, die neben anderen Reagenzien als Kontrollproben einen monoklonalen Antikörper enthalten.

EP 0 810 230 A2

**Beschreibung**

Die vorliegende Erfindung betrifft neue monoklonale Antikörper gegen Antigene aus HIV, insbesondere gegen das Antigen gp41 aus HIV-Subtyp 0 und das Antigen gp32 aus HIV II. Weiterhin betrifft die vorliegende Erfindung die Verwendung der Antikörper in einem immunologischen Verfahren, insbesondere als Kontrollproben in Testkits zum Nachweis von Anti-HIV-Antikörpern und zur Qualitätskontrolle bei der Herstellung von HIV-Antigenen für solche Testkits. Außerdem werden Testkits zum Nachweis von Antikörpern gegen mikrobielle Erreger, insbesondere virale Erreger offenbart, die neben anderen Reagenzien als Kontrollproben einen monoklonalen Antikörper enthalten.

Der Nachweis von Immunglobulinen in Körperflüssigkeiten, insbesondere in Humanseren, wird in der Diagnostik von Infektionen mit Mikroorganismen, insbesondere Viren, wie etwa HIV, Hepatitis-Viren etc. verwendet. Das Vorhandensein von spezifischen Immunglobulinen in der untersuchten Probe wird üblicherweise durch Reaktion mit einem oder mehreren Antigenen, die mit den spezifischen Immunglobulinen reagieren, nachgewiesen. Als Antigene können in solchen Tests z.B. rekombinante Polypeptidantigene oder synthetische Peptidantigene verwendet werden.

Verfahren zur Bestimmung von spezifischen Immunglobulinen müssen zuverlässig sein, um falsch positive oder negative Resultate zu vermeiden. Daher ist es erforderlich, daß sowohl die Hersteller als auch die Anwender von Testkits zum Nachweis von Antikörpern gegen mikrobielle Erreger aufwendige Qualitätskontrollen hinsichtlich der in den Testkits enthaltenen Nachweisantigene durchführen müssen. Für diese Qualitätskontrollen wurden bisher Humanseren verwendet, von denen bekannt war, daß sie die nachzuweisenden Antikörper enthalten. Ein Nachteil der als Kontrollproben verwendeten Humanseren besteht jedoch darin, daß die Aufbereitung aufgrund der Notwendigkeit einer Sterilisation sehr umständlich ist. Außerdem kann eine Standardisierung der Humanseren nicht ohne weiteres erreicht werden. Weiterhin sind Humanseren, die Antikörper gegen bestimmte mikrobielle Erreger enthalten, oft nicht in genügender Menge für Kontrollproben über mehrere Jahre verfügbar.

Die der Erfindung zugrunde liegende Aufgabe bestand somit darin, neue Mittel zur Qualitätskontrolle von immunologischen Testkits für den Nachweis von Antikörpern gegen mikrobielle Erreger bereitzustellen, bei denen die Nachteile des Standes der Technik mindestens teilweise beseitigt sind. Insbesondere sollen die Kontrollproben auf einfache Weise herstellbar, leicht standardisierbar und in großen Mengen verfügbar sein.

Es wurde nun gefunden, daß diese Aufgabe durch Verwendung von monoklonalen Antikörpern als Kontrollproben gelöst werden kann. Insbesondere wurde festgestellt, daß monoklonale Antikörper hervorragend zur Qualitätssicherung in Testkits zum Nachweis von Antikörpern gegen mikrobielle Erreger, insbesondere Viren, z.B. HIV, geeignet sind. Dieser Befund ist in höchstem Maße überraschend, da man aufgrund der bekannten hohen Variabilität von HI-Viren nicht erwarten konnte, daß monoklonale Antikörper als Kontrollproben die Zuverlässigkeit und Genauigkeit von Tests gewährleisten können. Gegenüber den bisher verwendeten Humanseren zeichnen sich die erfindungsgemäßen monoklonalen Antikörper weiterhin durch eine verbesserte Reproduzierbarkeit und durch eine leichtere Handhabbarkeit (keine Infektiosität) aus.

Ein erster Aspekt der vorliegenden Erfindung betrifft einen monoklonalen Antikörper der Klasse IgM, der gegen ein Antigen aus einem HIV gerichtet ist. Der Begriff "HIV" im Sinne der vorliegenden Erfindung umfaßt Human Immundefizienz-Viren, insbesondere HIV Typ I und HIV Typ II und Subtypen davon, z.B. HIV Subtyp 0.

Monoklonale Antikörper der Klasse IgM gegen ein HIV-Antigen sind insbesondere hervorragend als Serumimitate zur frühzeitigen Erkennung von Serokonversionen geeignet.

Vorzugsweise ist der monoklonale Antikörper gegen das Antigen gp41 aus HIV I und besonders bevorzugt gegen das Antigen gp41 aus HIV Subtyp 0 gerichtet ist. Ein weiterer bevorzugter Antikörper ist gegen das Antigen gp32 aus HIV II gerichtet.

Besonders bevorzugt sind die Antikörper gegen die Cystein-Loops der Antigene gp41 von HIV I bzw. HIV Subtyp 0 oder gp32 von HIV II gerichtet.

Ein Antikörper gegen gp41 aus HIV Subtyp 0 ist somit besonders bevorzugt gegen ein Peptidepitop mit der Aminosäuresequenz (I):

L S L W G C K G K L V C Y T S        (I)

gerichtet, wobei die Cystein-Reste gegebenenfalls eine Cystinbrücke ausbilden können. Beispiele für bevorzugte monoklonale Antikörper der Klasse IgM, die gegen gp41 aus HIV Subtyp 0 gerichtet sind, werden ausgewählt aus der Gruppe bestehend aus den Antikörpern 11.4.32 (DSM ACC 2266), 10.14.24 (DSM ACC 2265) und 10.12.25 (DSM ACC 2264) sowie Antikörpern mit äquivalenten Bindungseigenschaften, d.h. Antikörpern, die dasselbe Peptidepitop mit einer äquivalenten Bindungsaffinität erkennen.

Ein erfindungsgemäßer Antikörper gegen gp32 aus HIV II ist vorzugsweise gegen ein Peptidepitop mit der Aminosäuresequenz (II):

N S W G C A F R Q V C H T T        (II)

gerichtet, wobei die Cystein-Reste gegebenenfalls eine Cystinbrücke ausbilden können. Besonders bevorzugt werden die erfindungsgemäßen Antikörper ausgewählt aus der Gruppe bestehend aus den Antikörpern 2.6.6 (DSM ACC 2262), 2.11.7 (DSM ACC 2261) und 2.22.8 (DSM ACC 2260) sowie Antikörpern mit äquivalenten Bindungseigenschaften.

Ein erfindungsgemäßer Antikörper gegen gp41 von HIV I ist vorzugsweise gegen ein Peptidepitop mit der Aminosäuresequenz (III):

L G I W G C S G K L I C T T A V        (III)

gerichtet, wobei die Cysteinreste gegebenenfalls eine Cystinbrücke ausbilden können.

Ein weiterer bevorzugter Antikörper gegen gp41 von HIV I ist gegen ein Peptidepitop mit der Aminosäuresequenz (IV):

A V E R Y L K D Q Q L L G I W        (IV)

gerichtet.

Ein zweiter Aspekt der vorliegenden Erfindung betrifft monoklonale Antikörper, die gegen das Antigen gp41 aus einem HIV, insbesondere aus HIV I oder HIV Subtyp 0 gerichtet sind. Ein gegen gp41 aus HIV Subtyp 0 gerichteter Antikörper ist vorzugsweise gegen ein Peptidepitop mit der bereits zuvor angegebenen Aminosäuresequenz (I) gerichtet. Ein bevorzugter Antikörper gegen gp41 aus HIV I ist vorzugsweise gegen ein Peptidepitop mit der Aminosäuresequenz (III) gerichtet.

Die erfindungsgemäßen Antikörper weisen vorzugsweise die Klasse IgG oder IgM auf. Beispiele für bevorzugte Antikörper der Klasse IgM wurden bereits genannt. Bevorzugte Antikörper der Klasse IgG sind ausgewählt aus der Gruppe bestehend aus den Antikörpern 3.28.11 (DSM ACC 2269), 3.15.10 (DSM ACC 2268) und 3.6.5. (DSM ACC 2267) sowie Antikörpern mit äquivalenten Bindungseigenschaften.

Erfindungsgemäße Antikörper der Klasse IgG haben vorzugsweise eine Affinitätskonstante für das Antigen von $\geq 10^8$ $M^{-1}$ und besonders bevorzugt von $\geq 10^9$ $M^{-1}$.

Noch ein weiterer Aspekt der vorliegenden Erfindung betrifft monoklonale Antikörper, die gegen das Antigen gp32 aus HIV II gerichtet sind. Vorzugsweise sind diese Antikörper gegen ein Peptidepitop mit der zuvor angegebenen Aminosäuresequenz (II) gerichtet. Diese Antikörper können beispielsweise die Klassen IgG oder IgM aufweisen. Beispiele für bevorzugte Antikörper der Klasse IgM wurden bereits genannt. Bevorzugte Antikörper der Klasse IgG werden ausgewählt aus der Gruppe bestehend aus den Antikörpern 23.1.7 (DSM ACC 2263) und 20.5.3. (DSM ACC 2259) sowie Antikörpern mit äquivalenten Bindungseigenschaften.

Erfindungsgemäße Antikörper der Klasse IgG haben vorzugsweise eine Affinitätskonstante für das Antigen von $\geq 10^8$ $M^{-1}$ und besonders bevorzugt $\geq 10^9$ $M^{-1}$.

Die Herstellung erfindungsgemäßer Antikörper kann nach der Methode von Köhler und Milstein oder einer Weiterentwicklung davon erfolgen. Als Immunogene können Peptide, Polypeptide oder Viruslysate eingesetzt werden. Bevorzugte Immunogene sind rekombinante Polypeptide und trägergekoppelte Peptide, wobei als Träger beispielsweise Rinderserumalbumin (RSA) oder Keyhole Limpet Hämocyanin (KLH) verwendet werden können.

Zur Herstellung der besonders bevorzugten Antikörper der Klasse IgM kann das von Cianfriglia et al. (Hybridoma 2, 1983, 451-457) publizierte Imunisierungsschema angewendet werden. Auf die Offenbarung in dieser Literaturstelle wird ausdrücklich bezug genommen.

Auch Fragmente und Derivate der zuvor beschriebenen monoklonalen Antikörper sind ein Gegenstand der vorliegenden Erfindung. Solche Antikörperfragmente oder -derivate zeichnen sich dadurch aus, daß sie äquivalente Bindungseigenschaften wie ein vollständiger Antikörper besitzen, sich von diesem aber in Bereichen, die nicht für die Antigenerkennung wesentlich sind, unterscheiden. Antikörperfragmente sind herstellbar durch proteolytische Spaltung von vollständigen Antikörpern, wobei beispielsweise Fab-, Fab'- und F(ab')$_2$-Antikörperfragmente erhalten werden. Andererseits können Antikörperfragmente oder -derivate auch durch rekombinante DNA-Techniken hergestellt werden. Auf diese Weise lassen sich die verschiedensten Antikörperfragmente oder- derivate herstellen, z.B. einzelkettige Antikörperfragmente.

Erfindungsgemäße Antikörper, Antikörperfragmente und Antikörperderivate können auch mit einer Nachweisgruppe gekoppelt werden. Beispiele für geeignete Nachweisgruppen sind Lumineszenzgruppen wie etwa fluoreszierende Gruppen oder elektrochemilumineszenzfähige Metallchelate, Radiomarkierungen, Enzyme, Metallpartikel, NMR-aktive Gruppen etc. Die Kopplung solcher Markierungen an Antikörper ist dem Fachmann auf dem Gebiet der Immunologie bekannt und braucht daher nicht näher erläutert zu werden.

Die erfindungsgemäßen Antikörper, Antikörperfragmente und -derivate können in immunologischen Verfahren eingesetzt werden. Eine erste bevorzugte Anwendung ist die Verwendung von Kontrollproben in Testkits, die zum Nachweis von Anti-HIV-Antikörpern dienen. In solchen Testkits können die erfindungsgemäßen Antikörper einzeln oder in Kombination als positive Kontrolle statt des bisher verwendeten Humanserums eingesetzt werden. Die Vorteile der erfindungsgemäßen Antikörper gegenüber dem Humanserum sind die einfachere Standardisierbarkeit und die leichtere Verfügbarkeit. Überraschenderweise wurde festgestellt, daß die erfindungsgemäßen monoklonalen Antikörper eine ausreichende Sensitivität und Zuverlässigkeit als positive Kontrollproben besitzen.

Erfindungsgemäße Antikörper der Klasse IgM eignen sich besonders gut als Serumimitate und können daher die Früherkennung von Serokonversionen bei Anti-HIV-Tests verbessern.

Weiterhin sind die erfindungsgemäßen monoklonalen Antikörper als Kontrollproben bei der Herstellung von Testkits zum Nachweis von Anti-HIV-Antikörpern geeignet. Durch Einsatz der erfindungsgemäßen Antikörper können Antigenchargen getestet und deren Eignung für diagnostische Tests festgestellt werden.

Schließlich können die erfindungsgemäßen monoklonalen Anti-HIV-Antikörper auch als Detektionsantikörper zum Nachweis infektiöser Partikel oder als Immunadsorbentien zur Aufreinigung von Antigenen eingesetzt werden.

Noch ein weiterer Aspekt der vorliegenden Erfindung betrifft die generelle Verwendung von monoklonalen Antikörpern als Kontrollproben in Tests zum Nachweis von Antikörpern gegen mikrobielle Erreger, ausgewählt aus der Gruppe bestehend aus Pilzen, Mykoplasmen, Bakterien und Viren. Auch die Verwendung von monoklonalen Antikörpern als Kontrollproben bei der Herstellung von Testkits zum Nachweis von Antikörpern gegen mikrobielle Erreger ist Gegenstand der Erfindung.

Vorzugsweise werden monoklonale Antikörper als Kontrollproben zum Nachweis von Antikörpern gegen Viren, vorzugsweise HIV- oder Hepatitis-Viren, besonders bevorzugt HIV- oder Hepatitis-C-Viren und am meisten bevorzugt HIV-Viren eingesetzt.

Schließlich betrifft die Erfindung auch noch einen Testkit zum Nachweis von Antikörpern gegen mikrobielle Erreger, welcher dadurch gekennzeichnet ist, daß er neben anderen Nachweisreagenzien als Kontrollprobe einen monoklonalen Antikörper oder ein Fragment davon enthält. Ein Testkit zum Nachweis von Anti-HIV-Antikörpern enthält als Kontrollprobe vorzugsweise einen erfindungsgemäßen monoklonalen Antikörper wie vorstehend definiert oder ein Fragment davon.

Weiterhin soll die Erfindung durch die im nachfolgenden angegebenen Sequenzprotokolle und Beispiele erläutert werden.

Es zeigen SEQ ID NO. 1-4 die Aminosäuresequenzen der bevorzugten Peptidepitope (I) bis (IV).

**Beispiel 1:**

**Herstellung von Thiol-aktivierten Peptiden**

Teilsequenzen von HIV-Virusproteinen wurden mittels Fluorenylmethyloxycarbonyl-(Fmoc)-Festphasenpeptidsynthese an einem Batch-Peptidsynthesizer, z.B. von Applied Biosystems A431 oder A433, hergestellt. Dazu wurden jeweils 4.0 Äquivalente von folgenden Fmoc-Aminosäurederivaten verwendet:

| A | Fmoc-Ala-OH |
|---|---|
| C | Fmoc-Cys(Trt)-OH |
| D | Fmoc-Asp(tBu)-OH |
| E | Fmoc-Glu(tBu)-OH |
| F | Fmoc-Phe-OH |
| G | Fmoc-Gly-OH |
| H | Fmoc-His(Trt)-OH |
| I | Fmoc-Ile-OH |
| K | Fmoc-Lys(Phenylacetyl)-OH |
| L | Fmoc-Leu-OH |
| M | Fmoc-Met-OH |
| N | Fmoc-Asn(Trt)-OH |
| P | Fmoc-Pro-OH |
| Q | Fmoc-Gln(Trt)-OH |
| R | Fmoc-Arg(Pmc)-OH |
| S | Fmoc-Ser(tBu)-OH |
| T | Fmoc-Thr(tBu)-OH |
| U | Fmoc-βAlanin |
| V | Fmoc-Val-OH |
| W | Fmoc-Trp-OH |
| X | Boc-Lys(Fmoc)-OH |
| Y | Fmoc-Tyr(tBu)-OH |
| Z | Fmoc-Aminocapronsäure |

Die Aminosäuren oder Aminosäurederivate wurden in N-Methylpyrrolidon gelöst. Das Peptid wurde an 400-500 mg 4-(2′,4′-Dimethoxyphenyl-Fmoc-Aminomethyl)-Phenoxy-Harz (Tetrahedron Letters 28 (1987), 2107) mit einer Beladung von 0.4 - 0.7 mmol/g aufgebaut (JACS 95 (1973), 1328). Die Kupplungsreaktionen wurden bezüglich Fmoc-Aminosäurederivat mit 4 Äquivalenten Dicyclohexylcarbodiimid und 4 Äquivalenten N-Hydroxybenzotriazol in Dimethylformamid als Reaktionsmedium während 20 min durchgeführt. Nach jedem Syntheseschritt wurde die Fmoc-Gruppe mit 20 %igem Piperidin in Dimethylformamid in 20 min abgespalten. Enthalten die Peptide eine intramolekulare Disulfidbrücke, so wurde die Fmoc-geschützte Peptidsequenz vor der Kupplung des artifiziellen Spacers mit Jod in Hexafluorisopropanol/Dichlormethan (Kober et al., The Peptide Academic Press, New York, 1981, pp 145-47) an der Festphase oxidiert und anschließend die N-terminale Fmoc-Schutzgruppe abgespalten, und der Spacer sowie das N-terminale Cystein gekuppelt.

Die Freisetzung des Peptides vom Syntheseharz und die Abspaltung der säurelabilen Schutzgruppen - mit Ausnahme der Phenylacetylschutzgruppe am Lysin - erfolgte mit 20 ml Trifluoressigsäure, 0.5 ml Ethandithiol, 1 ml Thioanisol, 1.5 g Phenol und 1 ml Wasser in 40 min bei Raumtemperatur. Die Reaktionslösung wurde anschließend mit 300 ml gekühltem Diisopropylether versetzt und zur vollständigen Fällung des Peptides 40 min bei 0°C gehalten. Der Niederschlag wurde abfiltriert, mit Diisopropylether nachgewaschen, in 50%iger Essigsäure gelöst und lyophilisiert. Das erhaltene Rohmaterial wurde mittels präparativer HPLC an Delta-PAK RP C18-Material (Säule 50 x 300 mm, 100 Å, 15 μ) über einen entsprechenden Gradienten (Eluent A: Wasser, 0.1 % Trifluoressigsäure, Eluent B: Acetonitril, 0.1 % Trifluoressigsäure) in ca 120 min aufgereinigt. Die Identität des eluierten Materials wurde mittels Ionenspray-Massenspektrometrie geprüft.

**Beispiel 2:**

**Immunogensynthese**

Für die Verwendung als Immunogene wurden die entsprechenden Peptide mit einer reaktiven Mercaptofunktion, z.B. durch Einführen von einem zusätzlichen Cystein, hergestellt. Das Peptid kann dabei entweder N-,C-terminal oder auch beliebig in der Sequenz mit einem sogenannten Linker modifiziert sein. Die Synthese erfolgte wie in Beispiel 1 beschrieben.

Für die Umsetzung zum Immunogen wurde der $NH_2$-Gruppen-haltige Träger, vorzugsweise KLH oder RSA, zuerst mit dem entsprechenden Maleinimidoalkyl-Aktivester, durch Behandlung vorzugsweise mit Maleinimidohexyl- (MHS) oder Maleinimidopropyl-N-hydroxysuccinimidoester (MPS), beladen. Dadurch werden die Aminogruppen der $\varepsilon$-Aminoseitenkette von Lysinresten im Protein in Maleinimidgruppen umgewandelt.

Die Umsetzung des Trägers mit den Aktivestern erfolgte vorzugsweise in 0.1 M Kaliumphosphatpuffer pH 7.0 - 8.5 und einer Konzentration von 5-20 mg/ml innerhalb von 2-4 h bei Raumtemperatur. Die niedermolekularen Bestandteile wurden entweder über Dialyse oder Gelchromatographie (AcA 202-Gel, Eluent 0.1 M Kaliumphosphatpuffer pH 7-8.5) abgetrennt.

In einem weiteren Schritt wurde dann das Peptid mit der reaktiven Mercaptofunktion an das MHS-modifizierte Trägerprotein in 0.1 M Kaliumphosphatpuffer pH 7.0 innerhalb von 6 h bei Raumtemperatur gekuppelt. Nicht umgesetztes Peptid wurde entweder mittels Dialyse oder Gelchromatographie abgetrennt.

**Beispiel 3:**

**Immunisierung von Mäusen**

1. Immunisierung von Mäusen um MAK der Klasse **IgG** zu erhalten

1.1. **gp41-HIV-Subtyp 0 und gp41 HIV I**

12 Wochen alte Balb/c-Mäuse wurden mit 100 $\mu$g gp 41-Subtyp 0-Peptid (oxidierte Form, Sequenz LSLWCKGKLVCYTS mit einer Cystinbrücke zwischen den beiden Cysteinresten) bzw. gp41 HIV I-Peptid (oxidierte Form, Sequenz LGIWGCSGKLICTTAV mit einer Cystinbrücke zwischen den beiden Cysteinresten oder Sequenz AVERYLKDQQLLGIW), jeweils an RSA (Rinderserumalbumin) als Trägerprotein gekoppelt, in Gegenwart des Adjuvans CFA (Komplettes Freund'sches Adjuvans) intraperitoneal erstimmunisiert. Nach 6 Wochen folgten 3 weitere Immunisierungen intraperitoneal in monatlichen Abständen. Dabei wurden jeder Maus 100 $\mu$g Peptid-RSA zusammen mit IFA (Inkomplettem Freund'schen Adjuvans) verabreicht. Anschließend erfolgten die letzten beiden Immunisierungen intravenös mit je 100 $\mu$g Peptid-RSA in PBS-Puffer am 3. und 2. Tag vor der Fusion.

1.2. **gp 32 HIV II**

In diesem Falle wurde das soeben beschriebene Immunisierungsschema mit einem rekombinanten gp32 Fusionsprotein (siehe WO 94/12631) durchgeführt.

2. Immunisierung von Mäusen um MAK der Klasse **IgM** zu erhalten

Um MAKs der Klasse IgM zu erhalten, wurde das von Cianfriglia et al. (Hybridoma, Vol. 2 Nr. 4, 1983, Seite 451-457) publizierte Immunisierungsschema - sowohl für Subtyp 0 und HIV I gp41 als auch gp32 - angewendet.

Dieses Immunisierungsschema war wie folgt:

Tag 15 vor der Fusion:     100 $\mu$g Peptid in CFA, intraperitoneal
Tag 8 vor der Fusion:      100 $\mu$g Peptid in CFA, intraperitoneal
Tag 3 vor der Fusion:      400 $\mu$g Peptid in PBS-Puffer, intraperitoneal
Tag 2 vor der Fusion:      200 $\mu$g Peptid in PBS-Puffer,
                           intraperitoneal
                           plus
                           200 $\mu$g Peptid in PBS-Puffer, intravenös
Tag 1 vor der Fusion:      (wie Tag 2)
Tag O = Fusionstag

### Beispiel 4:

### Fusion und Klonierung

Die Fusion von Milzzellen der gemäß Beispiel 3 immunisierten Mäuse mit Myelomzellen erfolgte in Anlehnung an Galfré, Methods in Enzymology 73, 1981, 3. Dabei wurden ca. $1 \times 10^8$ Milzzellen der immunisierten Maus mit $2 \times 10^7$ Myelomzellen (P3X63-Ag8-653, ATCC CRL1580) gemischt und abzentrifugiert (10 min bei 300 g und 4°C). Die Zellen wurden dann einmal mit RPMI-1640-Medium ohne fötalem Kälberserum (FKS) gewaschen und bei 400 g in einem 50 ml Spitzröhrchen erneut abzentrifugiert. Der Überstand wurde abgekippt, das Zellsediment durch Klopfen leicht aufgelockert, 1 ml PEG (Molekulargewicht 4000, Merck, Darmstadt) dazugegeben, und durch Pipettieren gemischt. Nach 1 min im Wasserbad bei 37°C wurden bei Raumtemperatur 5 ml RPMI 1640 ohne FKS in einem Zeitraum von 4 - 5 min zugetropft, durchmischt auf 50 ml mit Medium (RPMI 1640 + 10% FKS) aufgefüllt und anschließend 10 min bei 400 g und 4°C zentrifugiert. Die sedimentierten Zellen wurden in RPMI 1640 Medium mit 10% FKS aufgenommen und in Hypoxanthin-Azaserin Selektionsmedium (100 mmol/l Hypoxanthin, 1 µg/ml Azaserin in RPMI 1640 + 10% FKS) eingesät. Als Wachstumsfaktor wurde dem Medium Interleukin-6 (100 U/ml) zugegeben.

Nach ca. 10 Tagen wurden die Primärkulturen auf spezifische Antikörperproduktion gegen gp41-Peptide oder gp32-Peptide getestet (siehe Beispiel 6). Antigen-positive Primärkulturen wurden mittels Durchflußcytometer (Facstar, Fa. Becton Dickinson) in 96er-Zellkulturplatten kloniert. Hierbei wurde dem Medium als Wachstumszusatz Interleukin-6 (100 U/l) zugegeben.

Auf diese Weise wurden folgende Zellinien erhalten, die bei der Deutschen Sammlung von Mikroorganismen und Zellkulturen GmbH, Mascheroder Weg 1b, D-38124 Braunschweig am 7. Mai 1996 gemäß den Bestimmungen des Budapester Vertrags hinterlegt wurden:

| Spezifität | Klon | Klasse | DSM-Nr. |
|---|---|---|---|
| | | | |
| (gp41, Subtyp 0) | 3.28.11 | IgG | ACC 2269 |
| | 3.15.10 | IgG | ACC 2268 |
| | 3.6.5 | IgG | ACC 2267 |
| | | | |
| | 11.4.32 | IgM | ACC 2266 |
| | 10.14.24 | IgM | ACC 2265 |
| | 10.12.25 | IgM | ACC 2264 |
| | | | |
| (gp32) | 23.1.7 | IgG | ACC 2263 |
| | 20.5.3 | IgG | ACC 2259 |
| | | | |
| | 2.6.6 | IgM | ACC 2262 |
| | 2.11.7 | IgM | ACC 2261 |
| | 2.22.8 | IgM | ACC 2260 |
| | | | |

### Beispiel 5:

### Immunglobulingewinnung aus den Zellkulturüberständen

Die erhaltenen Hybridomzellen wurden mit einer Dichte von $1 \times 10^5$ Zellen pro ml in RPMI 1640-Medium mit 10% FKS eingesät und 7 Tage lang in einem Fermenter (Fa. Thermodux, Wertheim/Main, Modell MCS-104XL, Best.-Nr. 144-050) vermehrt. In dem Kulturüberstand wurden Konzentrationen von durchschnittlich 100 µg monoklonaler Antikörper je ml erreicht. Die Reinigung dieses Antikörpers aus dem Kulturüberstand erfolgte nach proteinchemisch üblichen Methoden (z.B. gemäß Methods in Enzymology 121 (1986), 587 - 695).

**Beispiel 6:**

**Bestimmung der Spezifität der produzierten Antikörper**

Um die Spezifität der Antikörper im Kulturüberstand der Hyridomzellen zu bestimmen, wurden Streptavidin-beschichtete Mikrotiterplatten (Fa. MicroCoat, Penzberg) mit 100 ng/ml biotinyliertem gp 41-Peptid oder 2,5 μg/ml biotinyliertem gp 32-Peptid in PBS plus 1% Crotein C beschichtet (100 μl/Loch, 10 min Inkubation bei RT unter Schütteln) und danach 1 x mit 0,9% NaCl/0,05% Tween 20 gewaschen.

Jeweils 100 μl der zu untersuchenden Antikörperlösung wurden in ein beschichtetes Loch gegeben und 1 h bei Raumtemperatur unter Schütteln inkubiert. Nach 3-maligem Waschen mit 0,9% Natriumchlorid/0,05% Tween 20 wurden zum Nachweis von gebundenem Antikörper aus der Probe jeweils 100 μl eines Peroxidase (POD)-markierten Fab-Fragments eines polyklonalen Antikörpers aus dem Schaf gegen Maus-Fcγ (Boehringer Mannheim GmbH, Ident.-Nr. 1047 523 entsprechend 20 mU/ml) zugegeben, 1 h bei Raumtemperatur unter Schütteln inkubiert und anschließend 3 x mit 0,9% Natriumchlorid/0,05% Tween 20 gewaschen. Um MAKs der Klasse IgM zu finden, wurde als Nachweisantikörper ein Kaninchen-IgG POD-Konjugat gegen Maus-IgM (Sigma, Kat.-Nr. A-8786, μ-Ketten-spezifisch) verwendet.

Schließlich wurden jeweils 100 μl/Loch ABTS® (Boehringer Mannheim GmbH, Kat.-Nr. 1204521 und 1204530) zugegeben und nach 30 min bei Raumtemperatur die Extinktion bei 450/490 nm in einem MR700 Microplate Reader der Firma Dynatech gemessen.

**Beispiel 7:**

**Immunologische Tests**

Die immunologische Bewertung (Beispiel 8 - 12) der in Beispiel 4 hergestellten Antikörper wurde analog dem Boehringer Mannheim Enzymun-Test® Anti-HIV 1+2 (Boehringer Mannheim GmbH, Kat.-Nr. 1 165 062) durchgeführt. Das Testprinzip war ein 2-Schritt-Sandwich-ELISA mit Streptavidin-Technologie. Jedoch wurden statt der Humanseren die verschiedenen monoklonalen Antikörper eingesetzt und mit den jeweils biotinylierten Antigenen vermessen. Die Detektion erfolgte über ein Anti-Maus IgG-Antikörper-POD-Konjugat (Boehringer Mannheim GmbH, Kat.-Nr. 1 431 323) oder ein gegen Maus IgM gerichtetes Kaninchenantikörper-POD Konjugat (Sigma, Kat.-Nr. A8786). Alle anderen Zusatzreagenzien, wie Waschlösung, Streptavidintubes, ABTS usw. wurden beibehalten. Die Messungen wurden an den Enzymun®-Analysen-Automaten ES 22 oder ES 600 von Boehringer Mannheim durchgeführt.

**Beispiel 8:**

**Epitopkartierung der monoklonalen Antikörper**

Für die Epitopkartierung wurden Peptide synthetisiert, bei denen jeweils eine Aminosäure der nativen Sequenz des Standardantigens durch ein D-Alanin ausgetauscht wird. Ansonsten enthielten alle Peptide den gleichen Spacer und waren N-terminal biotinyliert. Aus der Reaktivität der Antikörper, d.h. dem detektierten Signal, läßt sich schließen, welche Aminosäurereste für die Antikörperbindung essentiell sind bzw. welche vom Antikörper nicht erkannt werden. Die Ergebnisse bei den ⟨gp32⟩IgG MAK's wurden mit der Epitopkartierung von Humanseren verglichen. Bei den ⟨gp41(SubO)⟩IgG's, ⟨gp41(SubO)⟩IgM's und den ⟨gp32⟩IgM's konnten die Ergebnisse nicht mit Humanseren verglichen werden, da bisher keine Humanseren mit derartigen Antikörperspezies in größerem Umfang verfügbar sind.

**Ergebnisse:**

Eine Epitopmarkierung des monoklonalen Anti-gp32-IgG Antikörpers 23.1.7 (DSM ACC 2263) ergab folgende für die Antigenerkennung essentielle Aminosäuren der Aminosäuresequenz (II), wobei die Aminosäureposition in Klammern angegeben ist: W(3), C(5), A(6), F(7), R(8), C(11) sowie Q(9) und T(14) mit geringerer Bedeutung. Eine entsprechende Epitopkartierung für den monoklonalen Antikörper 20.5.3 (DSM ACC 2259) ergab als essentielle Aminosäuren C(5), A(6), F(7), R(8), Q(9), V(10) und C(11). Auch für 2 weitere monoklonale Anti-gp32-IgG-Antikörper wurde eine Epitopkartierung durchgeführt. Für den Antikörper 20.3.1 wurden als essentielle Aminosäuren C(5) bis R(8) und C(11) sowie Q(9) mit geringerer Bedeutung identifiziert. Für den Antikörper 23.4.8 wurden als essentielle Aminosäuren S(2) mit geringerer Bedeutung, C(5) bis C(11) sowie T(14) mit geringerer Bedeutung indentifiziert. Die als Kontrollen verwendeten Humanseren ergaben als essentielle Aminosäuren W(3), G(4), C(5), F(7), R(8), V(10) und 10(11).

Diese Ergebnisse zeigen, daß bei Aminosäuresequenz (II) die Aminosäuren C(5) bis Q(9) und C(11) für die Erkennung durch alle getesteten Antikörper essentiell waren. Weitere wichtige Aminosäuren sind S(2), W(3), V(10) und T(14).

Für den monoklonalen Anti-gp41(Sub 0)-IgG-Antikörper 3.6.5 (DSM ACC 2267) wurden aus der Aminosäuresequenz (I) folgende Aminosäuren als essentiell identifiziert: S(2), C(6), V(11), C12), Y(13) mit geringerer Bedeutung und S(15). Für den hinterlegten Antikörper 3.15.10 (DSM ACC 2268) wurden S(2), C(6), K(9), L(10), V(11), C(12), T(14) mit geringerer Bedeutung und S(15) als essentielle Aminosäuren identifiziert. Für den hinteriegten Antikörper 3.28.11 (DSM ACC 2269) wurden als essentielle Aminosäuren S(2), C(6), K(9), V(11), C(12), T(14) mit geringerer Bedeutung und S(15) identifiziert.

Auch für drei weitere Anti-gp41(Sub 0)-IgG-Antikörper wurde eine Epitopkartierung durchgeführt. Für den Antikörper 3.5.1 wurden als essentielle Aminosäuren S(2), C(6) und K(9) bis S(15) identifiziert. Für den Antikörper 3.12.8 wurden S(2), C(6), K(9) bis C(12), T(14) und S(15) identifiziert. Für den Antikörper 3.31.13 wurden S(2), C(6), V(11), C(12), T(14) und S(15) identifiziert.

Diese Ergebnisse zeigen, daß bei Aminosäuresequenz (I) die Aminosäuren S(2), C(6), V(11), C(12) und S(15) für die Erkennung durch alle getesteten Antikörper essentiell waren. Weitere wichtige Aminosäuren sind K(9), L(10) und T(14) und Y(13).

### Beispiel 9:

### Bestimmung der Affinitätkonstanten sowie der Geschwindigkeitskonstanten der Assoziation und Dissoziation der produzierten Antikörper

Die Bestimmung der Affinitätkonstanten sowie der Geschwindigkeitskonstanten der Assoziation und Dissoziation der produzierten Antikörper erfolgte mit dem BIAcore Sensor der Firma Pharmacia. Das Messprinzip beruht auf der "Surface Plasmon Resonance". Die Messung wurde auf einem Biosensor, dem sog. Sensorchip durchgeführt. Hierbei wurde auf der Oberfläche eines mit Streptavidin beladenen Sensorchips biotinyliertes gp32- bzw. gp41-Peptid gebunden und damit immobilisiert. Über diesen Sensorchip wurde eine Lösung des zu bestimmenden Antikörpers geleitet, wobei der Antikörper durch nichtkovalente Wechselwirkungskräfte an das immobilisierte gp32- bzw. gp41-Peptid gebunden wird. Dadurch erhöht sich die Massendichte auf der Oberfläche des Sensorchips, die vom Gerät in ein zum Ausmaß der Bindung proportionales Messignal umgewandelt wird. Aus der zeitlichen Änderung des Signals, dem Sensorgramm, lassen sich die Geschwindigkeitskonstanten der Assoziation und Dissoziation und daraus die Affinitätskonstante berechnen.

Die Antikörper lassen sich ohne Beeinträchtigung der an die Oberfläche über Streptavidin-Biotin gebundenen Peptide mit einfachen Mitteln wieder ablösen, so daß an demselben Sensorchip weitere Bindungsexperimente unter identischen Randbedingungen durchgeführt werden können.

Zur Bindung der biotinylierten gp32- bzw. gp41-Peptide an den mit Streptavidin beladenen Sensorchip (SA 5, Pharmacia Biosensor) wurde eine 10 µM Lösung der biotinylierten Peptide in 10 mM HEPES/3,4 mM EDTA/150 mM NaCl/pH 7,4 mit einer Flußrate von 5 µl/min über den Sensorchip geleitet.

Anschließend wurden die Antikörper zugegeben und die Geschwindigkeitskonstanten der Assoziation und der Dissoziation für die Bindung an die Peptide aus den Sensorgrammen mit Hilfe einer Software des Herstellers (BIAevaluation 2.1, Pharmacia Biosensor) berechnet. Die Affinitätskonstante berechnet sich nach $K_a = k_{on}/k_{off}$. Die so ermittelten Werte der erfindungsgemäßen Antikörper sind in der folgenden Tabelle zusammengefaßt.

| Spezifität | Klon | kon | koff | Ka |
|---|---|---|---|---|
| | | 1/M*s | 1/s | 1/M |
| MAK⟨gp32 ⟩IgG | 23.1.7 | 1.2 E +5 | 1.0 E -5 | 1.2 E +10 |
| MAK⟨gp32 ⟩IgG | 20.5.3 | 1.7 E +5 | 5.1 E -5 | 3.3 E +9 |
| MAK⟨gp41, Sub0 ⟩IgG | 3.28.11 | 5.3 E +4 | 1.5 E -5 | 3.6 E +9 |
| MAK⟨gp41, Sub0 ⟩IgG | 3.15.10 | 3.7 E +4 | 1.4 E -5 | 2.6 E +9 |
| MAK⟨gp41, Sub0 ⟩IgG | 3.6.5 | 4.0 E +4 | 1.4 E -5 | 2.9 E +9 |

**Beispiel 10:**

**Verwendung von monoklonalen Antikörpern zur Qualitätssicherung**

In der Qualitätssicherung wurden neuproduzierten Packungchargen jeweils mit verschiedenen monoklonalen Antikörper als Interne Kontrollproben (IPK) überprüft.

In der Tabelle sind Messungen mit Boehringer Mannheim Enzymuntest Anti-HIV 1+2+Subtyp O aufgeführt.

Als Kontrollproben wurden folgende monoklonalen Antikörper eingesetzt.

IKP-HIV 2 A = MAK ⟨gp32⟩IgG 20.3.1. in Humanserum verdünnt (c= 5 μg/ml)
IKP-HIV 2 B = MAK ⟨gp32⟩IgG 20.5.3. in Humanserum verdünnt (c= 0,2 μg/ml)
IKP-HIV 2 C = MAK ⟨gp32⟩IgG 23.1.7. in Humanserum verdünnt (c= 25 μg/ml)
IKP-SubO-A = MAK ⟨gp41SubO⟩IgG 3.31.13. in Humanserum verdünnt (c= 0,5 μg/ml)
IKP-SubO-B = MAK ⟨gp41SubO⟩IgG 3.6.5. in Humanserum verdünnt (c= 0,5μg/ml)
IKP-SubO-C = MAK ⟨gp41SubO⟩IgG 3.12.8. in Humanserum verdünnt (c= 0,2μg/ml)

| Packungschargenvergleichbarkeit | | | | |
|---|---|---|---|---|
| Probe | Referenz-Charge | Charge 1 | Charge 2 | Charge 3 |
| IKP-HIV 2 A | 2.21 | 1.94 | 2.85 | 2.19 |
| IKP-HIV 2 B | 9.76 | 9.94 | 11.3 | 12.22 |
| IKP-HIV 2 C | 0.95 | 0.92 | 1.08 | 0.97 |
| IKP-SubO-A | 7.85 | 6.07 | 8.08 | 0.03 |
| IKP-SubO-B | 7.94 | 6.18 | 8.51 | 0.03 |
| IKP-SubO-C | 2.91 | 2.25 | 2.64 | 0.04 |

Die Signale sind in cutoff-Indices angegeben.

Aus den Bewertungen geht hervor, daß die Produktion der Chargen 1 und 2 im Vergleich zur Referenzcharge in Ordung war. Produktionscharge 3 ist nicht tauglich, da die Subtyp O-Reaktivität fehlt.

**Beispiel 11:**

**Verwendung von monoklonalen Antikörpern zur Qualitätssicherung**

Die monoklonalen Antikörper können für die Entwicklung und Inprozeßkontrolle von sogenannten Polyantigenen (DE-A-44 30 972.4) verwendet werden. Die Bewertung erfolgte im Boehringer Mannheim Enzymun-Test Anti-HIV 1+2+Subtyp O wobei die Packungsantigene (Flasche 2a und 2b) gegen Einzelantigene z.B. gegen HIV-2-Antigene ausgetauscht wurden.

Als Kontrollproben wurden neben den in Beispiel 11 beschriebenen Proben IKP-HIV 2A, IKP-HIV 2B und IKP-HIV 2C folgende Proben eingesetzt:

HIV 2-IgM A = MAK ⟨gp32⟩IgM 2.6.6. in Humanserum verdünnt (c = 20 μg/ml)
HIV 2-IgM B = MAK ⟨gp32⟩IgM 2.11.7. in Humanserum verdünnt (c= 20 μg/ml)
HIV 2-IgM C = MAK ⟨gp32⟩IgM 2.22.8. in Humanserum verdünnt (c= 2 μg/ml)

| Chargenvergleichbarkeit von Einsatzstoffen | | | | |
|---|---|---|---|---|
| Probe | Referenz-Charge | HIV-2-Antigencharge 1 | HIV-2-Antigencharge 2 | HIV-2-Antigencharge 3 |
| IKP-HIV 2 A | 2.92 | 2.64 | 3.02 | 5.81 |
| IKP-HIV 2 B | 8.8 | 8.97 | 6.5 | 10.53 |
| IKP-HIV 2 C | 0.92 | 1.36 | 1.28 | 1.38 |
| HIV 2-IgM A | 11.92 | 4.92 | 10.26 | 21.92 |
| HIV 2-IgM B | 14.26 | 6.22 | 12.31 | 25.25 |
| HIV 2-IgM C | 2.44 | 1.24 | 2.43 | 4.99 |

Die Signale sind in cutoff-Indices angegeben.

Aus den Bewertungen geht hervor, daß Antigencharge 2 im Vergleich zur Referenzcharge in Ordung war. Antigencharge 1 ist nicht tauglich, da die IgM-Reaktivität zu gering ist, Antigencharge 3 ist jedoch eine Verbesserung gegenüber der Referenzcharge.

**Beispiel 12:**

**Inprozeßkontrolle bei der Synthese von Polyantigenen**

Mit Hilfe von monoklonalen Antikörpern, die spezifisch mit einem Epitop reagieren, kann die Funktionalität von Polyhaptenen beispielsweise im indirekten Testkonzept bewertet werden. Polyhaptene sind Antigene, die einen immunologisch mit dem zu bestimmenden Antikörper nicht reagierenden Träger, beispielsweise ein Peptid, Polypeptid oder einen synthetischen Träger, umfassen, an den mehrere Epitopbereiche gekoppelt sind. Geeignete Polyhaptene und Verfahren zu deren Herstellung sind in DE-A-44 30 972.4 offenbart.

Dazu wurden biotinylierte Polyhaptene (Herstellung gemäß Beispiel 2 von DE-A-44 30 972.4) mit einem Epitopspezifischen MAK im oben beschriebenen Testprinzip inkubiert.

Die Ergebnisse waren wie folgt:

| | Mak-Konz [μg/ml] | Referenz | Polyhapten Ch 01 | Polyhapten Ch 02 | Polyhapten Ch 03 |
|---|---|---|---|---|---|
| **Negativ-Serum** | | 0.000 | 0.000 | 0.000 | 0.010 |
| | | | | | |
| **MAK ⟨gp32⟩ IgG 20.5.3** | 0 | 0.000 | 0.010 | 0.003 | 0.030 |
| | 2 | 4.220 | 4.220 | 0.015 | 4.230 |
| | 5 | 7.250 | 7.260 | 0.030 | 7.270 |
| | 25 | 9.120 | 9.120 | 0.068 | 9.160 |
| | 50 | 9.180 | 9.170 | 0.073 | 9.230 |
| Alle Meßsignale in mE | | | | | |

Diese Ergebnisse Zeigen, daß die Polyhapten-Chargen 01 und 03 geeignet sind, Polyhapten-Charge 02 aber ungeeignet ist.

**Patentansprüche**

1. Monoklonaler Antikörper der Klasse IgM, der gegen ein Antigen aus einem HIV gerichtet ist.

2. Antikörper nach Anspruch 1,
**dadurch gekennzeichnet,**
daß er gegen das Antigen gp41 aus HIV Subtyp 0 gerichtet ist.

3. Antikörper nach Anspruch 2,
**dadurch gekennzeichnet,**
daß er gegen ein Peptidepitop mit der Aminosäuresequenz (I):
L S L W G C K G K L V C Y T S(I)
gerichtet ist, wobei die Cysteinreste gegebenenfalls eine Cystinbrücke ausbilden können.

4. Antikörper nach einem der Ansprüche 1 - 3,
ausgewählt aus der Gruppe bestehend aus den Antikörpern 11.4.32 (DSM ACC 2266), 10.14.24 (DSM ACC 2265) und 10.12.25 (DSM ACC 2264) sowie Antikörpern mit äquivalenten Bindungseigenschaften.

5. Antikörper nach Anspruch 1,
**dadurch gekennzeichnet,**
daß er gegen das Antigen gp32 aus HIV II gerichtet ist.

6. Antikörper nach Anspruch 5,
   **dadurch gekennzeichnet,**
   daß er gegen ein Peptidepitop mit der Aminosäuresequenz (II):
   N S W G C A F R Q V C H T T (II)
   gerichtet ist, wobei die Cysteinreste gegebenenfalls eine Cystinbrücke ausbilden können.

7. Antikörper nach einem der Ansprüche 1, 5 oder 6,
   ausgewählt aus der Gruppe bestehend aus den Antikörpern 2.6.6. (DSM ACC 2262), 2.11.7 (DSM ACC 2261) und 2.22.8 (DSM ACC 2260) sowie Antikörpern mit äquivalenten Bindungseigenschaften.

8. Antikörper nach Anspruch 1,
   **dadurch gekennzeichnet,**
   daß er gegen das Antigen gp41 aus HIV I gerichtet ist.

9. Antikörper nach Anspruch 8,
   **dadurch gekennzeichnet,**
   daß er gegen ein Peptidepitop mit der Aminosäuresequenz (III):
   L G I W G C S G K L I C T T A V        (III)
   gerichtet ist, wobei die Cysteinreste gegebenenfalls eine Cystinbrücke ausbilden können.

10. Antikörper nach Anspruch 8,
    **dadurch gekennzeichnet,**
    daß er gegen ein Peptidepitop mit der Aminosäuresequenz (IV):
    A V E R Y L K D Q Q L L G I W (IV)
    gerichtet ist.

11. Monoklonale Antikörper, der gegen das Antigen gp41 aus einem HIV gerichtet ist.

12. Antikörper nach Anspruch 11,
    **dadurch gekennzeichnet,**
    daß gegen das Antigen gp41 aus HIV I gerichtet ist.

13. Antikörper nach Anspruch 11,
    **dadurch gekennzeichnet,**
    daß er gegen das Antigen gp41 aus HIV Subtyp 0 gerichtet ist.

14. Antikörper nach Anspruch 13,
    **dadurch gekennzeichnet,**
    daß er gegen ein Peptideptitop mit der, Aminosäuresequenz (I):
    L S L W G C K G K L V C Y T S        (I)
    gerichtet ist, wobei die Cysteinreste gegebenenfalls eine Cystinbrücke ausbilden können.

15. Antikörper nach Anspruch 11, ausgewählt aus der Gruppe bestehend aus den Antikörpern 3.28.11 (DSM ACC 2269), 3.15.10 (DSM ACC 2268) und 3.6.5. (DSM ACC 2267) sowie Antikörpern mit äquivalenten Bindungseigenschaften.

16. Antikörper nach Anspruch 11,
    ausgewählt aus der Gruppe bestehend aus den Antikörpern 11.4.32 (DSM ACC 2266), 10.14.24 (DSM ACC 2265) und 10.12.25 (DSM ACC 2264) sowie Antikörpern mit äquivalenten Bindungseigenschaften.

17. Monoklonaler Antikörper, der gegen das Antigen gp32 aus HIV II gerichtet ist.

18. Antikörper nach Anspruch 17,
    **dadurch gekennzeichnet,**
    daß er gegen ein Peptidepitop mit der Aminosäuresequenz (II):
    N S W G C A F R Q V C H T T        (II)
    gerichtet ist, wobei die Cysteinreste gegebenenfalls eine Cystinbrücke ausbilden können.

19. Antikörper nach Anspruch 17, ausgewählt aus der Gruppe bestehend aus den Antikörpern 23.1.7 (DSM ACC 2263) und 20.5.3 (DSM ACC 2259) sowie Antikörpern mit äquivalenten Bindungseigenschaften.

20. Antikörper nach Anspruch 17,
ausgewählt aus der Gruppe bestehend aus den Antikörpern 2.6.6. (DSM ACC 2262), 2.11.7 (DSM ACC 2261) und 2.22.8 (DSM ACC 2260) sowie Antikörpern mit äquivalenten Bindungseigenschaften.

21. Konjugat, umfassend einen Antikörper nach einem der Ansprüche 1 - 20 oder ein Fragment oder Derivat davon und eine Nachweisgruppe.

22. Verwendung eines oder mehrerer monoklonaler Antikörper nach einem der Ansprüche 1 - 20 oder von Fragmenten oder Derivaten davon in einem immunologischen Verfahren.

23. Verwendung nach Anspruch 22 als Kontrollproben in Testkits zum Nachweis von Anti-HIV-Antikörpern.

24. Verwendung nach Anspruch 22 oder 23,
**dadurch gekennzeichnet,**
daß man monoklonale Antikörper der Klasse IgM zur Simulation der Früherkennung von Serokonversionen einsetzt.

25. Verwendung nach Anspruch 22 als Kontrollproben bei der Herstellung von Testkits zum Nachweis von Anti-HIV-Antikörpern.

26. Verwendung nach Anspruch 22 als Detektionsantikörper zum Nachweis infektiöser Partikel.

27. Verwendung nach Anspruch 22 als Immunadsorbentien zur Aufreinigung von Antigenen.

28. Verwendung von monoklonalen Antikörpern als Kontrollproben in Tests zum Nachweis von Antikörpern gegen mikrobielle Erreger, ausgewählt aus der Gruppe bestehend aus Pilzen, Mykoplasmen, Bakterien und Viren.

29. Testkit zum Nachweis von Antikörpern gegen mikrobielle Erreger,
**dadurch gekennzeichnet,**
daß er neben anderen Reagenzien als Kontrollprobe einen monoklonalen Antikörper oder ein Fragment davon enthält.

30. Testkit nach Anspruch 29 zum Nachweis von Anti-HIV-Antikörpern,
**dadurch gekennzeichnet,**
daß er als Kontrollprobe einen monoklonalen Antikörper nach einem der Ansprüche 1 - 20 oder ein Fragment davon enthält.

SEQUENZPROTOKOLL

(1) ALLGEMEINE ANGABEN:

    (i) ANMELDER:
        (A) NAME: Boehringer Mannheim GmbH
        (B) STRASSE: Sandhoferstr. 116
        (C) ORT: Mannheim
        (E) LAND: Germany
        (F) POSTLEITZAHL: 68305

    (ii) BEZEICHNUNG DER ERFINDUNG: Anti-HIV-Antikoerper als Kontrollproben

    (iii) ANZAHL DER SEQUENZEN: 4

    (iv) COMPUTER-LESBARE FASSUNG:
        (A) DATENTRÄGER: Floppy disk
        (B) COMPUTER: IBM PC compatible
        (C) BETRIEBSSYSTEM: PC-DOS/MS-DOS
        (D) SOFTWARE: PatentIn Release #1.0, Version #1.30 (EPA)

(2) ANGABEN ZU SEQ ID NO: 1:

    (i) SEQUENZKENNZEICHEN:
        (A) LÄNGE: 15 Aminosäuren
        (B) ART: Aminosäure
        (C) STRANGFORM: Einzelstrang
        (D) TOPOLOGIE: linear

    (ii) ART DES MOLEKÜLS: Peptid

    (xi) SEQUENZBESCHREIBUNG: SEQ ID NO: 1:

    Leu Ser Leu Trp Gly Cys Lys Gly Lys Leu Val Cys Tyr Thr Ser
    1            5                10             15

(2) ANGABEN ZU SEQ ID NO: 2:

    (i) SEQUENZKENNZEICHEN:
        (A) LÄNGE: 14 Aminosäuren
        (B) ART: Aminosäure
        (C) STRANGFORM: Einzelstrang
        (D) TOPOLOGIE: linear

    (ii) ART DES MOLEKÜLS: Peptid

    (xi) SEQUENZBESCHREIBUNG: SEQ ID NO: 2:

    Asn Ser Trp Gly Cys Ala Phe Arg Gln Val Cys His Thr Thr
    1            5                10

(2) ANGABEN ZU SEQ ID NO: 3:

    (i) SEQUENZKENNZEICHEN:
        (A) LÄNGE: 16 Aminosäuren
        (B) ART: Aminosäure
        (C) STRANGFORM: Einzelstrang
        (D) TOPOLOGIE: linear

    (ii) ART DES MOLEKÜLS: Peptid

    (xi) SEQUENZBESCHREIBUNG: SEQ ID NO: 3:

    Leu Gly Ile Trp Gly Cys Ser Gly Lys Leu Ile Cys Thr Thr Ala Val
    1         5         10        15

(2) ANGABEN ZU SEQ ID NO: 4:

    (i) SEQUENZKENNZEICHEN:
        (A) LÄNGE: 15 Aminosäuren
        (B) ART: Aminosäure
        (C) STRANGFORM: Einzelstrang
        (D) TOPOLOGIE: linear

    (ii) ART DES MOLEKÜLS: Peptid

    (xi) SEQUENZBESCHREIBUNG: SEQ ID NO: 4:

    Ala Val Glu Arg Tyr Leu Lys Asp Gln Gln Leu Leu Gly Ile Trp
    1         5         10        15